(19) Europäisches Patentamt
European Patent Office
Office européen des brevets

(11) **EP 4 545 062 A1**

(12) **EUROPEAN PATENT APPLICATION**

(43) Date of publication:
**30.04.2025 Bulletin 2025/18**

(21) Application number: **24460031.8**

(22) Date of filing: **15.10.2024**

(51) International Patent Classification (IPC):
*A61K 8/06* (2006.01)    *A61K 8/34* (2006.01)
*A61K 8/37* (2006.01)    *A61K 8/49* (2006.01)
*A61K 8/65* (2006.01)    *A61K 8/86* (2006.01)
*A61K 38/00* (2006.01)    *A61Q 19/00* (2006.01)

(52) Cooperative Patent Classification (CPC):
**A61K 8/65; A61K 8/068; A61K 8/345; A61K 8/375;
A61K 8/4973; A61K 8/86; A61K 38/00;
A61Q 19/00;** A61K 2800/21

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **23.10.2023 PL 44646023**

(71) Applicant: **Politechnika Gdanska
80-233 Gdansk (PL)**

(72) Inventors:
• **SZUMALA, Patrycja
82-10 STEGNA (PL)**
• **KOZLOWSKA-TYLINGO, Katarzyna
80-125 Gdansk (PL)**

(74) Representative: **Pawlowska-Bajerska, Justyna
Gdansk University of Technology
ul. G. Narutowicza 11/12
80-233 Gdansk (PL)**

(54) **TRANSDERMAL MICROEMULSION COMPOSITION COMPRISING LOW MOLECULAR WEIGHT COLLAGEN**

(57) Transdermal microemulsion composition with a high degree of the active compound penetration into the deeper layers of the skin, characterized by the fact that it comprises 20.7-21.5% by weight of oil, 32.1-33.3% by weight of surfactant, in proportion by weight at least 2:1, 16.2-16.7% by weight of cosurfactant, in proportion by weight at least 1:2 and 28.5-31% by weight of aqueous solution of low molecular weight collagen, and the biopolymer content in the microemulsion is 0.285-0.31% by weight of low molecular weight collagen with a molecular weight of 2-3 kDa.

EP 4 545 062 A1

## Description

The invention refers to transdermal microemulsion composition comprises a biologically active compound in the form of a biopolymer, i.e. low molecular weight collagen, showing a high degree of penetration into living skin layers. The prepared microemulsions are used as dermocosmetics or nanocarriers for transporting active ingredients or drugs applied to the skin.

Microemulsions are thermodynamically stable colloidal systems consisting of water phase droplets dispersed in an oil phase or oil phase droplets dispersed in an water phase. The size of these dispersed droplets is in the range of 10-150 nm. Droplets in microemulsions are stabilized by surfactants and cosurfactants. Many compounds can be used as active ingredients in the microemulsion systems, including vitamins, ceramides, peptides. However, not all studies confirm the effectiveness of microemulsions in delivering drugs or other active ingredients to the target site in the skin. It is particularly difficult to transport hydrophilic, highmolecular polymer compounds, such as collagen, whose molecular size is too large to cross the epidermal barrier of the skin.

Collagen is the main component of connective tissue, which gives the skin its strength and elasticity and is involved in the creation of new cells. It is one of the main structural components of the skin. With age and under the influence of e.g. UV radiation, collagen fibers become crosslinked, which leads to their fragility and defragmentation (a decrease in tensile and compressive strength). These changes lead to a decrease in skin density and elasticity, as well as excessive water loss and the formation of wrinkles.

Providing the skin with collagen from the outside, i.e. using simple cosmetic products with collagen, is very difficult, because the stratum corneum of the epidermis, i.e. the outermost layer of the skin, has a lipophilic character and is a barrier for hydrophilic, relatively large collagen molecules. Typical cosmetic products containing collagen do not therefore act transdermally, but only have a superficial moisturizing effect. In this case, the active compound does not penetrate the skin and acts only on the surface of the epidermis. The only currently effective method of introducing collagen is to use them in the form of soft tissue fillers and inject into a given part of the skin. However, injections are an invasive medical procedure, are painful, expensive, require the presence of a specialist and repetition within a given time frame. For this reason, other solutions and preparations are sought that would enable the delivery of this biopolymer to the deeper layers of the skin. A desirable solution would therefore be, to introduce collagen into a carrier, whose structure and specially selected formula would enable the penetration of this active ingredient through biological membranes.

The article Kupper S., Klosowska-Chomiczewska I., Szumata P., in the journal: Carbohydrate Polymers 175 (2017) 347-354, describes the physicochemical properties and composition of microemulsions containing as biologically active ingredients: low or high molecular weight collagen or hyaluronic acid in amounts ranging from 0 to 0.03-0.27 wt.% in product. The following compounds were used to obtain the product: deionized water or aqueous solutions of collagen or hyaluronic acid, oil, i.e. isopropyl myristate, surfactants, i.e. sorbitan oleate and ethoxylated (20) sorbitan oleate, cosurfactants, i.e. propylene glycol and ethanol. In this work, the structure of these microemulsions was studied, depending on the surfactants content and different concentrations of collagen and hyaluronate solutions used for their preparation. The stability, particle size and rheological properties of these microemulsions were also presented.

In the publication by Szumata P., Jungnickel Ch., Kozlowska-Tylingo K., Jacyna B. and Cal K., International Journal of Pharmaceutics 572 (2019) 118738, the above-described microemulsions with low molecular weight or high molecular weight collagen or hyaluronic acid were described in terms of transdermal action, i.e. the penetration of active ingredients, i.e. collagen and hyaluronate, from microemulsion through a synthetic membrane imitating the skin. The article showed different levels of collagen and hyaluronic acid penetration from the microemulsion, depending on the content of active ingredients and other components of the microemulsion, but the most effective systems contained medium amounts of a stabilizing mixture, i.e. surfactants and co-surfactants ($ME_{5/5}$).

The microemulsions described above comrises ethanol, which may irritate the skin. The article did not present an analysis of the safety of using these microemulsions on the skin, so their use as safe transdermal cosmetic products has not been confirmed. Ethanol used in the preparation of microemulsions influences on the formation of microemulsion systems, because ethanol creates a more flexible layer that stabilizes microemulsions, but also improves the solubility of active substances (the role of cosurfactant) in microemulsions. Moreover, this alcohol improves the action of microemulsions because it liquefies the intercellular cement of the skin, which is the main route of penetration of active ingredients into the skin. The loosening of the ordered structure of cement, in turn, affects the easier penetration and transport of ingredients to deeper layers of the skin. It is therefore advisable to replace ethanol with other compounds that do not cause irritation and fulfill the desired effects as cosurfactants.

According to the article by Neubert R. H. H., Sommer E., Schölzel M., Tuchscherer B., Mrestani Y. and Wohlrab J. published in the European Journal of Pharmaceutics and Biopharmaceutics 124 (2018) 28-33, microemulsions containing peptides are known, which after topical application, act on the surface of the epidermis. The microemulsion consisted of: 0.5% tetrapeptide dissolved in 10% water and butylene glycol (1:1 wt.), TEGO® Care PL4 (16.7%) and TEGO® SMO V (8.3%) as emulsifiers and 65% of oil (TEGOSOFT® P). The degree of penetration of tetrapeptides from the microemulsion and from the standard emulsion formulation in human skin *ex vivo* was examined. Relatively high amounts (40 to 58%) of tetrapeptide penetrated from the standard emulsion formulation to the epidermis, but the peptides did not reach the target compartment in the skin (living skin layers). Penetration of tetrapeptides from the microemulsion was more effective, but most of these peptides did not remain in the skin, but penetrated into the acceptor chamber.

The invention relates to provided transdermal microemulsion composition with a high degree of active compound penetration into the deeper layers of the skin, in an amount of at least 11.6% within 24 hours of application. According to the invention, the composition is characterized by the content of 20.7-21.5% by weight of oil - preferably isopropyl myristate, 32.1-33.3% by weight of surfactant, preferably sorbitan oleate and ethoxylated (20) sorbitan oleate, in a weight ratio of 2:1, 16.2-16.7% by weight of cosurfactant, preferably propylene glycol and 1,2-pentylene glycol in a weight ratio of 1:2, and 28.5-31% by weight of an aqueous solution of low molecular weight collagen, and the biopolymer content in the microemulsion is 0.285-0.31% by weight of low molecular weight collagen with a molecular weight of 2-3 kDa. The best effects are obtained by selecting the preferable components of oil, surfactant and cosurfactant - then microemulsions has high biopolymer transport capacity, rate. The selected 1:2 surfactant ratio allows obtaining microemulsions with effective properties, and the 1:2 co-surfactant ratio enables obtaining a high level of collagen transport. Preferably, the active compound is present in the water phase as a 1% water solution.

Experimental studies were conducted in order to find and select ingredients that would create a microemulsion safe for use on the skin, free from potentially irritating ingredients such as ethanol, and demonstrating a significant level of penetration of the active ingredients "encapsulated" in these microemulsions into the deeper layers of the skin. The so-called penetration enhancers in microemulsions (surfactants, cosurfactants, lipids) selected according to the invention in quantitative and qualitative terms and matters lead to liquefaction and/or disruption of the lipid bilayer structure of the epidermis, leading to increased percutaneous penetration of the active compound. An important provision is not to use ethanol as a penetration enhancer, in order to form a safe microemulsion, but with a high degree of transdermal penetration of the active compound.

Instead of ethanol, glycols were used, thanks to which the microemulsion is a dermocosmetic or a product with a nanocarrier structure for the safe transport of active ingredients and drugs. Glycols, including propylene glycol and pentylene glycol, have a similar effect to ethanol. The use of such glycols mixture instead of ethanol resulted in higher "encapsulation" (introduction) of collagen water solution in the microemulsion, as compared to the microemulsion with ethanol.

As a result of the conducted experimental studies performed to provide the compositon, formulas of microemulsion systems - receipe, mixture with low molecular weight collagen were selected.

The invention refers to pharmaceutical-cosmetic product for administration of a biologically active compound, the advantage of which is the possibility of transporting collagen to deeper, living parts of the skin, where this biopolymer occurs naturally (dermis). In addition, the microemulsions according to the invention do not comprises ethanol, do not exhibit irritating properties and do not exhibit cytotoxic activity in relation to the skin. This product can therefore be safely applied to the skin. The product is a nanocarrier that crosses the skin barrier and allows biopolymer molecules to penetrate deeper layers of the skin, is nonirritating and additionally improves the skin moisture. The transdermal composition of the microemulsion, according to the invention, is characterized by a high degree of penetration of the active compound into deeper layers of the skin, at the level of at least 11.6% within 24 hours of application.

The invention is presented in the following examples.

Example 1.

Research was conducted on the development of a cosmeticpharmaceutical microemulsion for topical 'application, the composition of which shows the most effective transdermal penetration of biologically active ingredients, i.e. collagen in low-molecular form. For this reason, many microemulsion recipes were tested in order to develop the best one for use as a drug or dermocosmetic, i.e. for transporting collagen to the deeper layers of the skin. Tables 1 and 2 list selected microemulsion components tested to develop the invention.

Table 1. Microemulsion ingredients, given in grams, comprising collagen - description of the obtaining method

| Active compound | | | Low molecular weight collagen |
|---|---|---|---|
| Ingredients of tested microemulsions (grams) | isopropyl myristate (oil) | | 3 |
| | sorbitan oleate : ethoxylated sorbitan oleate (2:1 wt.) (surfactants) | | 4,66 |
| | propylene glycol: 1,2-pentylene glycol (1:2 wt.) (cosurfactants) | | 2,34 |
| | active compound | | 0,045 |
| | deionized water | | 4,455 |
| Amount of 1% aqueous solution of active compound used (grams) | | | 4,5 |
| Transdermal penetration (%) | | | 11,6 |

Table 2. Microemulsion components given in weight percentage, comprising collagen

| Active compound | | | Low molecular weight collagen |
|---|---|---|---|
| Ingredients of tested microemulsions (grams) | isopropyl myristate (oil) | | 20,7 |
| | sorbitan oleate : ethoxylated sorbitan oleate (2:1 wt.) (surfactants) | | 32,1 |
| | propylene glycol: 1,2-pentylene glycol (1:2 wt.) (cosurfactants) | | 16,2 |
| | active compound | | 0,31 |
| | deionized water | | 30,69 |
| Amount of 1% aqueous solution of active compound used (grams) | | | 31 |
| Transdermal penetration (%) | | | 11,6 |

The preparation of all microemulsions is as follows: appropriate amounts (given in Tables 1 and 2) of isopropyl myristate, sorbitan oleate, ethoxylated (20) sorbitan oleate, propylene glycol and 1,2-pentylene glycol are added to a beaker. The ingredients are mixed using a mechanical stirrer (300 rpm) until uniform. Then, a 1% aqueous solution of the active compound is added dropwise. After adding the entire solution, the microemulsions are mixed for an additional 5 minutes. A 1% solution was prepared according to the ingredients given in Table 1 to finally obtain the established amount of the active compound in the microemulsion, which is given in Table 2.

All 1% aqueous solutions of active compounds, i.e. low molecular weight collagen, were obtained in the same way, i.e.: the calculated amount of the active compound (e.g. 0.1 gram) was weighed on an analytical balance into a glass vessel with a cap. Then, the calculated amount of deionized water (e.g. 9.9 grams) was weighed in the same vessel. The active compound with water in the vessel was closed with a cap and placed in a refrigerator for 24 hours to swell and dissolve. After this time, the solution was removed from the refrigerator and mixed with a magnetic stirrer (300 rpm) at room temperature for 15 minutes.

The method of obtaining the tested microemulsions consisted in mixing appropriate amounts of oil, surfactants and co-surfactants, and then adding a 1% aqueous collagen solution to the mixture, in such an amount to finally obtain the desired concentration. The entire process was carried out at room temperature. The obtained tested microemulsions comprises 20.7-21.5% by weight of oil - isopropyl myristate, 32.1-33.3% by weight of surfactants - sorbitan oleate and ethoxylated (20) sorbitan oleate (in a weight ratio of 2:1), 16.2-16.7% by weight of cosurfactants, i.e. propylene glycol and 1,2-pentylene glycol (in a weight ratio of 1:2) and 28.5-31% by weight of the aqueous collagen solution. The biopolymer content in the microemulsions tested is 0.285-0.31 wt.% of low molecular weight collagen (the product is described as comprising 28.5-31 milligrams of collagen). The droplet size of the resulting microemulsions tested is between 60 and 80 nanometers.

Under *in vitro* conditions (in the Franz chamber, using a synthetic, multilayer Strat-M membrane, which imitates the structure of human skin), the penetration of collagen from the above described composition of microemulsions is 11.6% within 24 hours of application to the membrane.

In vitro transdermal penetration studies were conducted using a Franz cell. The microemulsions studied, presented in Tables 1 and 2, in a finite dose of approximately 25-40 mg, were placed on a synthetic, multilayer Strat-M membrane (effective diffusion surface area was 0.25 cm3) located in the donor part. The acceptor part of the chamber contained water. During the entire experiment, the temperature of the diffusion chamber was maintained at 37 °C. After 24 h from the application of the microemulsion to the membrane, the collagen content in the acceptor chamber was determined ($\mu$g/ml). The transdermal penetration value was calculated using the following formula (Cavalcanti, A.L.M., Reis, M.Y.F.A., Silva, G.C.L., Ramalho, I.M.M, Guimarães, G.P., Silva, J.A., Saraiva, K.L.A., Damascene, B.P.G.L. (2016) Microemulsion for topical application of pentoxifylline: In vitro release and in vivo evaluation, International Journal of Pharmaceutics, 506, 351-360):

$$\text{Transdermal penetration (\%)} = C \cdot 100/A,$$

where:

C - concentration of the active compound in the acceptor fluid after 24 h ($\mu$g/ml),
A - concentration of the active compound in the microemulsion applied to the membrane ($\mu$g/ml).

Based on the study of many microemulsion formulas, microemulsions with the highest degree of transdermal penetration were selected, i.e. the composition specified in the tables 1 and 2. This composition can be used as a transdermal microemulsion in dermocosmetics or medicines, i.e. a product for transporting collagen to the deeper layers of the skin.

As a result of developing a quantitatively and qualitatively composition, the obtained microemulsions, according to the invention, demonstrate the ability of transdermal transport of the biopolymer to the deeper layers of the skin - low molecular weight collagen. Dermatological tests performed confirm that the produced microemulsions belong to nonirritating products. The study of the TEWL parameter (transepidermal water loss level) after application of the developed microemulsions to the skin confirmed that they reduce the TEWL level of the skin, i.e. have a positive effect on the hydration and condition of the skin - table 3. The described properties and action confirm that the invention can be used as a safe dermocosmetic or nanocarrier of active ingredients and drugs for skin applications.

Table 3. Increase in skin hydration level after application of the obtained microemulsions

| Microemulsion name | Biopolymer in microemulsion | TEWL value [%] |
|---|---|---|
| Transdermal microemulsion | LMW collagen | - 14,77 |

Example 2: Microemulsion with the highest level of active compound penetration.

20.7 wt.% (3 g) of isopropyl myristate, 21.4 wt.% (3.11 g) of sorbitan oleate, 10.7 wt.% (1.56 g) of ethoxylated (20) sorbitan oleate, 5.4 wt.% (0.78 g) of propylene glycol and 10.8 wt.% (1.56 g) of 1,2-pentylene glycol are added to the beaker. The ingrediencies are mixed using a mechanical stirrer (300 rpm) until uniform. Then 31 wt.% (4.5 g) of a 1% aqueous solution of low molecular weight collagen is added dropwise. After adding the collagen solution, the microemulsion is mixed for 5 minutes. The microemulsion of the given composition shows a transdermal penetration of low molecular weight collagen at the level of 11.6 $\pm$ 1.7% after 24 hours of application to the membrane. This result indicates the potential amount of low molecular weight collagen that would penetrate the living layer of the skin (below the epidermis) after 24 hours of application of the product to the skin.

**Claims**

1. Transdermal microemulsion composition with a high degree of active compound penetration into deeper skin layers, comprising oil, water, at least one surfactant, at least one cosurfactant and a biologically active compound in the form of a biopolymer being low molecular weight collagen, is characterized that it comprises 20.7-21.5 wt.% of oil, 32.1-33.3 wt.% of surfactant, in a weight ratio of at least 2:1, 16.2-16.7 wt.% of cosurfactant in a weight ratio of at least 1:2 and 28.5-31 wt.% of an aqueous solution of low molecular weight collagen, and the biopolymer content in the microemulsion is 0.285-0.31 wt.% of low molecular weight collagen with a molecular weight of 2-3 kDa.

2. The composition according to claim 1, wherein the collagen is present in the aqueous phase as a 1% aqueous solution.

3. The composition according to claim 1-2, wherein it comprises 20.7-21.5 wt.% of isopropyl myristate as oil.

4. The composition according to claim 1-3, wherein it comprises 32.1-33.3 wt.% of surfactants: sorbitan oleate and ethoxylated sorbitan oleate, in a weight ratio of 1:2.

5. the composition according to claim 1-4, wherein it comprises 16.2-16.7 wt.% of cosurfactants in the form of propylene glycol and 1,2-pentylene glycol, in a weight ratio of 1:2

Europäisches
Patentamt

European
Patent Office

Office européen
des brevets

# EUROPEAN SEARCH REPORT

Application Number

EP 24 46 0031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | SZUMALA PATRYCJA ET AL: "Transdermal transport of collagen and hyaluronic acid using water in oil microemulsion", INTERNATIONAL JOURNAL OF PHARMACEUTICS, ELSEVIER, AMSTERDAM, NL, vol. 572, 6 November 2019 (2019-11-06), XP085937760, ISSN: 0378-5173, DOI: 10.1016/J.IJPHARM.2019.118738 [retrieved on 2019-11-06] | 1-4 | INV. A61K8/06 A61K8/34 A61K8/37 A61K8/49 A61K8/65 A61K8/86 A61K38/00 A61Q19/00 |
| Y | * Points 1 to 3.1 and 4 * | 5 | |
| Y | WO 2013/142247 A1 (PREC DERMATOLOGY INC [US]) 26 September 2013 (2013-09-26) * claims 8, 9, 14 * | 5 | |
| A | CN 103 599 067 A (YANCHENG INST TECHNOLOGY) 26 February 2014 (2014-02-26) * claims; examples * | 1-5 | |
| A | CN 103 071 149 A (ZHEJIANG HAILISHENG GROUP CO LTD ET AL.) 1 May 2013 (2013-05-01) * paragraph [0042] * * claims 2, 5; table 1 * | 1-5 | TECHNICAL FIELDS SEARCHED (IPC) A61K A61Q |

The present search report has been drawn up for all claims

| Place of search | Date of completion of the search | Examiner |
|---|---|---|
| Munich | 10 March 2025 | Perrone Dunet, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 46 0031

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2025

| Patent document cited in search report | Publication date | Patent family member(s) | Publication date |
|---|---|---|---|
| WO 2013142247 A1 | 26-09-2013 | AU 2013235483 A1 | 09-10-2014 |
| | | BR 112014023379 A2 | 20-06-2017 |
| | | CA 2867877 A1 | 26-09-2013 |
| | | CN 104363893 A | 18-02-2015 |
| | | EP 2827842 A1 | 28-01-2015 |
| | | JP 2015510934 A | 13-04-2015 |
| | | KR 20140134712 A | 24-11-2014 |
| | | US 2013251644 A1 | 26-09-2013 |
| | | US 2013253014 A1 | 26-09-2013 |
| | | US 2013253015 A1 | 26-09-2013 |
| | | WO 2013142247 A1 | 26-09-2013 |
| | | WO 2013142249 A1 | 26-09-2013 |
| | | WO 2013142251 A1 | 26-09-2013 |
| CN 103599067 A | 26-02-2014 | NONE | |
| CN 103071149 A | 01-05-2013 | NONE | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KUPPER S.** ; **KLOSOWSKA-CHOMICZEWSKA I.** ; **SZUMATA P.** *journal: Carbohydrate Polymers*, 2017, vol. 175, 347-354
- **SZUMATA P.** ; **JUNGNICKEL CH.** ; **KOZLOWSKA-TYLINGO K.** ; **JACYNA B.** ; **CAL K.** *International Journal of Pharmaceutics*, 2019, vol. 572, 118738
- **NEUBERT R. H. H.** ; **SOMMER E.** ; **SCHÖLZEL M.** ; **TUCHSCHERER B.** ; **MRESTANI Y.** ; **WOHLRAB J**. *European Journal of Pharmaceutics and Biopharmaceutics*, 2018, vol. 124, 28-33
- **CAVALCANTI, A.L.M.** ; **REIS, M.Y.F.A.** ; **SILVA, G.C.L.** ; **RAMALHO, I.M.M** ; **GUIMARÃES, G.P.** ; **SILVA, J.A.** ; **SARAIVA, K.L.A.** ; **DAMASCENE, B.P.G.L.** Microemulsion for topical application of pentoxifylline: In vitro release and in vivo evaluation. *International Journal of Pharmaceutics*, 2016, vol. 506, 351-360